# EUROPEAN PATENT APPLICATION

(11) **EP 4 364 762 A1**
(43) Date of publication of application: **08.05.2024**
(21) Application number: 22205317.5
(22) Date of filing: 03.11.2022
(51) Int. Cl.: A61M 1/06

(54) **A SYSTEM FOR APPLYING A VACUUM TO A BREAST AND A BREAST PUMP USING THE SAME**

(71) Applicant: Koninklijke Philips N.V., 5656 AG Eindhoven (NL)
(72) Inventor: VAN DEN BIJGAART, Adrianus Wilhelmus Dionisius Maria, Eindhoven (NL); PALERO, Jonathan Alambra, Eindhoven (NL)
(74) Representative: Philips Intellectual Property & Standards

(57) **Abstract**

A wearable system for applying a vacuum to breast of a user comprises first and second elastically deformable membranes for positioning over each other and over the breast, with the first (inner) membrane defining a milk collection volume over the breast. The first membrane is manually deformable by actuating the second (outer) membrane, and a valve releases air from the milk collection volume. A pressure reduction is thereby generated when the first membrane tries returns to its default shape. A space between the first and second membranes is vented to the surrounding atmosphere to allow the second membrane to return to its default shape without generating a reduction in pressure. The second membrane functions as a protecting outer layer for the first membrane.

## Description

### FIELD OF THE INVENTION

This invention relates to the application of a vacuum to a breast, in particular as part of a breast pump.

### BACKGROUND OF THE INVENTION

Breast pumps are used by breast feeding women to extract milk from their breast such that the extracted milk can be fed to their babies at a later time.

It is well known that the best nutrition for babies is breast milk. The world health organization (WHO) recommends to breast feed babies for at least one year, preferably longer. However, mothers often go back to work after only several weeks or months. To provide the best nutrition to their babies, mothers may then express milk using a breast pump. The expressed milk can be stored and given to the baby at a later stage and/or by somebody else.

To use a breast pump, the breast is typically placed into a funnel-shaped cup and a vacuum is applied such that milk is extracted. A motorized breast pump typically has two operating modes, namely a stimulation mode and an extraction mode. During the stimulation mode, the milk ejection reflex is stimulated. When the breast pump is activated, a pressure source such as a vacuum pump inside the breast pump generates a vacuum and the stimulation mode can be started. The vacuum pump is typically driven by an electric motor. For portable breast pumps, these motors are driven by battery power.

There are also more simple breast pumps that generate a vacuum mechanically and manually instead of using an electric motor. This provides a lower cost device.

Wearable breast pumps are characterized in that they can be fully worn on the user's body, allowing the user to move around freely. Some of these are small enough to fit into a user's bra.

This invention relates in particular to wearable breast pumps with manual (mechanical) pressure reduction. The simplest way to generate and maintain a vacuum in this type of device is by using an elastically deformable element which can be operated directly by hand by deforming the element and then releasing the element. There is then a bias caused by the elasticity of the deformable element it to restore to the original shape, and this results in the generation of a vacuum.

The deformed element however results in an undesirable shape. Disturbance of the restoring process to the original shape (due to external forces) is also a problem desirable, as it influences the vacuum performance. This disturbance may for example be caused by wearing of a bra over the breast pump.

There is a need for a simply mechanical wearable device that generates and maintains a vacuum while preventing the device from being exposed to disturbance forces when in use (e.g., when wearing a bra).

### SUMMARY OF THE INVENTION

The invention is defined by the claims.

According to examples in accordance with the invention, there is provided a wearable system for applying a vacuum to breast of a user, comprising:
a first, elastically deformable, membrane for positioning over the breast, for defining a milk collection volume over the breast;
a second, elastically deformable, membrane positioned over the first membrane; and
a one-way valve for allowing release of air from the milk collection volume,
wherein:
   the first membrane is manually deformable to reduce the size of the milk collection volume such that an elastic restoring force generates a reduction in pressure,
   the second membrane is manually deformable to induce the manual deformation of the first membrane; and
   a space between the first and second membranes is vented to the surrounding atmosphere to allow the second membrane to return to its default shape without generating a reduction in pressure.

This system enables a reduction in pressure (i.e. partial vacuum) to be applied to the breast by manually actuating (e.g. squeezing) a membrane, in particular to reduce the volume of a space over the breast. During the reduction in volume, air is allowed to escape. When the actuation (e.g. squeezing) force is removed, the elastic restoring force results in a partial vacuum (i.e. an under-pressure relative to the surrounding ambient pressure) in the milk collection volume, which is used to assist the expression of milk.

The first membrane is actuated by means of the second membrane. However, the second membrane is allowed to return elastically to its original shape because the space between the two membranes is vented. The second (outer) membrane thus provides a protective covering to the first (inner) membrane and thereby prevents accidental release of the generated vacuum, for example caused by clothing of the user. It also returns to a desired outer shape so that the external shape gives rise to a desired outer appearance (i.e. the shape formed by the clothing of the user).

The one-way valve is for example integrated into the first flexible membrane. This reduces the needed number of components.

The second membrane is preferably stiffer than the first membrane. In this way, it is more resistant to external forces than the first membrane, but it is still sufficiently soft that a user can deform (e.g. squeeze) the second membrane to make it change shape and accordingly actuate the first membrane.

The second membrane is however preferably sufficiently stiff to resist deformation when interacting with clothing worn by the user of the system when the system is in use.

The first membrane is for example formed of silicone.

The second membrane is for example formed of silicone.

The two membranes may be the same material, e.g. with different thickness or other shape characteristics to result in different stiffnesses, or they may be different materials (e.g. different silicones or different materials altogether).

The invention also provides a wearable manual breast pump comprising:
the pressure application system defined above; and
a milk collection container for receiving milk from the milk collection volume.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
- Fig. 1: shows a known breast pump system;
- Fig. 2: shows a wearable breast pump;
- Fig. 3: shows in schematic form a breast pump using a pressure generation system of the invention; and
- Fig. 4: shows how the milk collection system generates a vacuum to the breast.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a wearable system for applying a vacuum to breast of a user, comprising first and second elastically deformable membranes for positioning over each other and over the breast, with the first (inner) membrane defining a milk collection volume over the breast. The first membrane is manually deformable by actuating the second (outer) membrane, and a valve releases air from the milk collection volume. A pressure reduction is thereby generated when the first membrane tries to return to its default shape. A space between the first and second membranes is vented to the surrounding atmosphere to allow the second membrane to return to its default shape without generating a reduction in pressure. The second membrane functions as a protecting outer layer for the first membrane. The milk collection system is for example part of a breast pump.

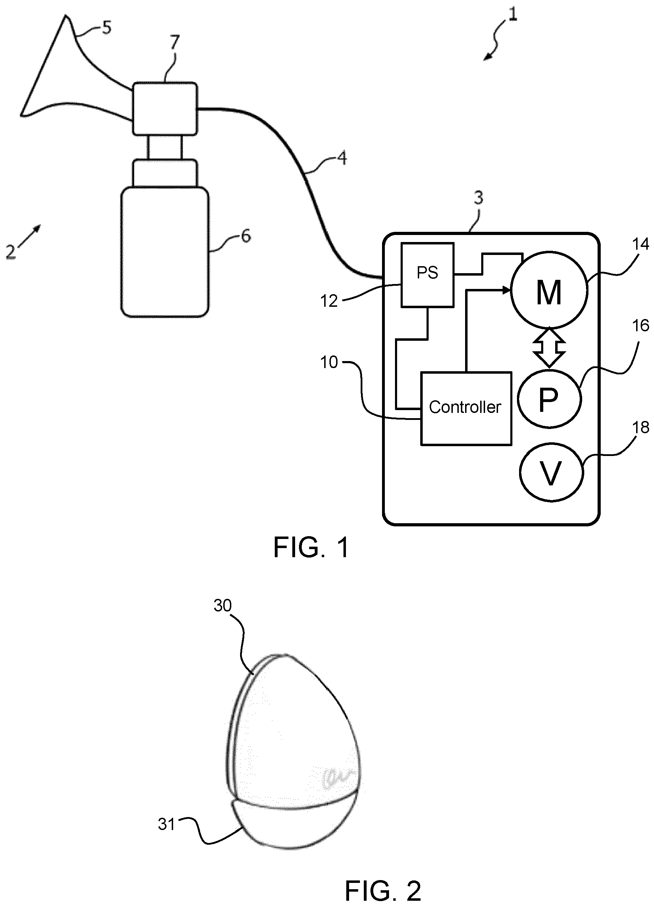

Fig. 1 shows a known breast pump system 1, comprising an expression unit 2 and a drive arrangement for controlling the expression function of the breast pump.

In the example shown, the drive arrangement comprises a pump arrangement 3 which is connected via a tube 4 to the expression unit 2. The pump arrangement includes various components in addition to a pump impeller and the pump motor, so may be considered to be a general operating unit.

The expression unit 2 is formed with a main body 7, a funnel 5 (known as a breast shield) for receiving a breast of a user and a receptacle 6 for collecting the expressed milk. The funnel 5 and the receptacle 6 are connected to the main body 7. The main body 7 comprises a vacuum chamber. A flexible membrane known as the diaphragm is located in the vacuum chamber. The diaphragm prevents expressed milk from flowing into the tube 4 leading to the pump arrangement unit 3.

The operating unit, including the pump arrangement 3, may instead be directly mounted and connected to the main body 7. In this case, the membrane prevents expressed milk from flowing directly into the pump arrangement 3.

The pump arrangement 3 comprises a controller 10, a power source 12, a motor 14 and a vacuum pump 16 (i.e. the impeller driven by the motor 14). The controller controls 10 the operation of the power source 12, motor 14 and vacuum pump 16. The pump arrangement 3 further comprises a solenoid valve 18.

In use, the vacuum pump 16 applies a vacuum to the membrane located in the main body 7 so that it deforms. The membrane deforms to create a vacuum in the funnel 5 which in turns applies a vacuum to the breast which enables milk to be expressed.

The vacuum is applied to the breast at intervals. That is, a pressure differential is applied on a cyclic basis. After a vacuum has been established, the pressure from the vacuum is released by the use of the solenoid valve which is temporarily opened. The solenoid valve is an electromechanically operated valve configured to open and close an air passage that connects to the vacuum side of the vacuum pump to ambient air such that when the solenoid valve is closed, the vacuum pump generates a vacuum in the expression unit which enables milk to be expressed from the breast of a user. When the solenoid valve is opened, the vacuum generated by the vacuum pump is released as ambient air flows towards the vacuum or negative pressure created by the vacuum pump such that the pressure exerted on the breast of a user is partially or completely reduced.

This is a basic description of the known operation of a standard breast pump system.

There are also wearable breast pumps, which are for example for mounting within a feeding bra. In some examples, all of the drivetrain components described above may then be formed within an outer enclosure which fits over the breast. A top part contains the drivetrain (pump, motor, controller) and a bottom part forms the collection vessel. This enables breast pumping to be performed more discretely.

There are also simpler mechanical breast pumps, in which the pressure is applied manually. Instead of a cyclic pressure, a static manually generated pressure is applied to the breast. The pressure may be reset periodically (for example the filling of a milk container with expressed milk may result in a loss of the applied pressure).

This invention relates in particular to wearable breast pumps using manual pressure generation.

Fig. 2 shows a wearable breast pump, comprising an expression kit 30 attached to a respective milk collection vessel 31. There are also other types of wearable breast pump whereby the expression unit is fixed to a breast and the pump unit and/or milk container are situated elsewhere on the body of a user.

The invention relates in particular to the way a reduced pressure is manually generated, and thus relates more generally to a wearable system for applying a vacuum to breast of a user.

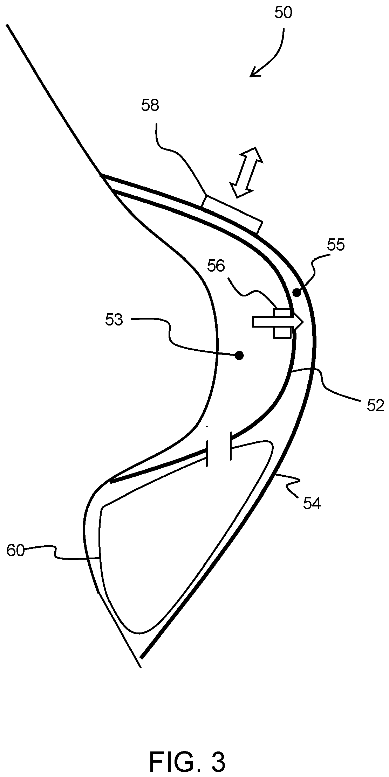

Fig. 3 shows in schematic form a breast pump using a pressure reduction system of the invention.

The breast pump 50 comprises a first, elastically deformable, membrane 52 for positioning over the breast, for defining a milk collection volume 53 over the breast.

A second, elastically deformable, membrane 54 is positioned over the first membrane 52, and a space 55 is defined between them.

A one-way valve 56 allows release of air from the milk collection volume 53 to the ambient surroundings, either directly or via the space 55 between the membranes (as shown in Fig. 3). The one-way valve 56 prevents the milk collection volume 53 filling with air. Thus, if the volume is reduced, for example by squeezing the membrane 52, the reduction in volume is held by the valve 56 (assuming the first membrane is sealed over the breast). The elasticity of the first membrane then delivers a restoring force and this creates a partial vacuum in the milk collection volume, i.e. a vacuum applied to the breast.

The space 55 between the membranes is vented to the atmosphere as shown by venting 58. The venting 58 may simply be a series of holes or slits, for example positioned over the whole area rather than in one place, so that the risk of the venting being blocked by clothing is avoided. The venting allows two-way air flow, so that the pressure on opposite sides of the second membrane is equalized. After any deformation of the second membrane 54, it can return to its default shape without generating a reduction in pressure.

In use, the first membrane 52 is manually deformed to reduce the size of the milk collection volume 53 and the elastic restoring force then generates a reduction in pressure as explained above. The user does not however engage the first membrane 52. Instead, the second membrane 54 is manually deformable, and is manually actuated by the user, to induce the desired manual deformation of the first membrane 52.

Thus, a reduction in pressure is applied to the breast by manually actuating (e.g. squeezing) the outer second membrane 54, and this in turn squeezes the first membrane 52 to reduce the size (i.e. volume) of the milk collection volume 53. When the squeezing force is removed, the vacuum is used to assist the expression of milk.

The second membrane returns elastically to its original shape and it provides a protective covering to the first membrane. It prevents accidental release of the generated vacuum, for example caused by clothing of the user.

The one-way valve 56 may be integrated into the first membrane 52, for example formed as a flap valve using the material of the membrane. Alternatively it may be a separate component around which, or to which, the membrane is fitted.

The second membrane is preferably stiffer than the first membrane so that it is more resistant to external forces than the first membrane. It needs to be sufficiently soft that a user can deform (e.g. squeeze) the second membrane to make it change shape and accordingly actuate the first membrane. However, it also needs to be sufficiently stiff to resist deformation when interacting with clothing worn by the user of the system when the system is in use (e.g. a bra into which the wearable breast pump is fitted).

The first and/or second membranes maybe formed of silicone. The materials may be the same, with the differences in mechanical characteristics created by the geometric configuration, e.g. the use of strengthening ribs or different material thickness. Alternatively, the two membranes may be formed from different materials (e.g. different silicones or different elastic materials altogether).

The milk collection container 60 may be an integral part of the first membrane 52 or it may be a separate part (as shown in Fig. 3) connected to the milk collection volume 53. The milk collection container may be flexible or rigid.

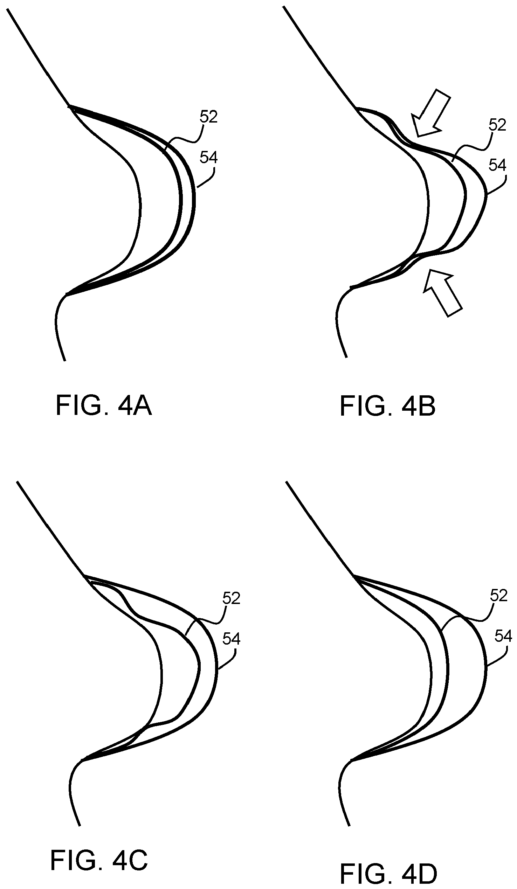

Fig. 4 shows how the vacuum is generated. The milk collection container and valve/venting are removed to make the images simpler.

Fig. 4A shows the first and second membranes 52, 54 with their default shapes. When deformed, they generate an elastic restoring force to return to these default shapes.

Fig. 4B shows a force applied to the second membrane 52 by a user, e.g. a squeezing force. The squeezing may be up-down or side-to-side depending on the design. The force may even be applied to the milk collection container, for example when the first membrane and the milk collection container are formed as a single integral component. Both membranes are deformed, and the second (outer) membrane is transferring its deformation to the first (inner) membrane.

Fig. 4C shows the second membrane returned to its default shape after the squeezing force is removed. This is because there is no remaining external force or pressure differential across the membrane.

Fig. 4D shows that the first membrane will adopt a shape which does not retain the exact shape as when it was being squeezed, because the internal stresses and strains in the membrane will become balanced. However, the size (i.e. volume) of the milk collection volume is reduced.

In the example above, the milk container is directly attached to the remainder of the breast pump to form a wearable unit. The invention may however be applied to designs where the milk container is mounted somewhere else on the body, connected by a milk tube.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

## Claims

1. A wearable system for applying a vacuum to breast of a user, comprising:
a first, elastically deformable, membrane (52) for positioning over the breast, for defining a milk collection volume (53) over the breast;
a second, elastically deformable, membrane (54) positioned over the first membrane; and
a one-way valve (56) for allowing release of air from the milk collection volume,
wherein:
the first membrane (52) is manually deformable to reduce the size of the milk collection volume such that an elastic restoring force generates a reduction in pressure,
the second membrane (54) is manually deformable to induce the manual deformation of the first membrane; and
a space (55) between the first and second membranes is vented to the surrounding atmosphere to allow the second membrane to return to its default shape without generating a reduction in pressure.

2. The system of claim 1, wherein the one-way valve (56) is integrated into the first flexible membrane.

3. The system of claim 1 or 2, wherein the second membrane (54) is stiffer than the first membrane (52).

4. The system of claim 3, wherein the second membrane (54) is sufficiently stiff to resist deformation when interacting with clothing worn by the user of the system when the system is in use.

5. The system of any one of claims 1 to 4, wherein the first membrane (52) is formed of silicone.

6. The system of any one of claims 1 to 5, wherein the second membrane (54) is formed of silicone.

7. A wearable manual breast pump comprising:
the system of any one of claims 1 to 6; and
a milk collection container (60) for receiving milk from the milk collection volume.
